# FASCICULE DE BREVET EUROPEEN

(11) **EP 0 558 395 B1**
(45) Date de publication et mention de la délivrance du brevet: **23.04.1997**
(21) Numéro de dépôt: 93400454.0
(22) Date de dépôt: 22.02.1993
(51) Int. Cl.: C07C 237/46, A61K 49/04, C07C 215/10, C07C 215/12, C07D 207/12

(54) **Composés utilisables dans des produits de contraste pour la radiographie**
Verbindungen verwendbar als Kontrastmittel für Radiographie
Compounds useful in contrast agents for radiography

(30) Priorité: 24.02.1992 FR 9202112
(43) Date de publication de la demande: 01.09.1993
(73) Titulaire: GUERBET S.A., F-93430 Villepinte (FR)
(72) Inventeur: Bennani, Fatima-Zarha, F-75014 Paris (FR); Le Greneur, Soizic, F-91440- Bures-Sur-Yvette (FR); Simonot, Christian, F-75020 Paris (FR); Meyer, Dominique, F-94100 Saint-Mauer (FR)
(74) Mandataire: Le Guen, Gérard

(56) Documents cités:
- EP-A- 0 049 745
- EP-A- 0 082 803
- EP-A- 0 185 130
- EP-A- 0 437 144
- DE-A- 3 429 949
- CHEMICAL ABSTRACTS, vol. 88, no. 4, 23 janvier 1978, Columbus, Ohio, US; abrégé no. 24102c, T. NAKANISHI, "Hydrophilic elastomers", page 50, colonne 1; & JP-A-51 038 756
- CHEMICAL ABSTRACTS, vol. 114, no. 7, 18 février 1991, Columbus, Ohio, US; abrégé no. 62573v, A. DEFOIN et al, "Synthesis of aminoerythrose and of aminoerythritol derivatives via Diels-Alder cycloadditions of 1-tert- butyldimethylsiloxybutadiene with acylnitrose dienophiles", page 738, colonne 2

## Description

La présente invention concerne un composé utilisable dans des produits de contraste pour la radiographie.

On utilise depuis longtemps comme produits de contraste des composés iodobenzéniques présentant sur le noyau benzénique plusieurs atomes d'iode, en général 3 atomes d'iode par noyau benzénique, et divers autres substituants. Ces autres substituants sont des groupes pharmacologiquement acceptables qui permettent l'administration des composés chez l'homme et les animaux. Ces substituants sont d'une manière générale choisis, d'une part, pour conférer aux composés une hydrosolubilité suffisante en vue de l'administration de ces composés en solution aqueuse et, d'autre part, pour conférer à ces composés une tolérance suffisante par l'organisme humain.

A cet effet, on a proposé des structures non-ioniques, c'est-à-dire des dérivés iodobenzéniques possédant des substituants non-ioniques.

La présente invention vise à fournir un nouveau composé non-ionique qui soit bien toléré par l'organisme humain, stable en solution aqueuse, qui présente une bonne solubilité dans l'eau et qui en solution aqueuse présente une faible viscosité.

Le composé de l'invention a pour formule :

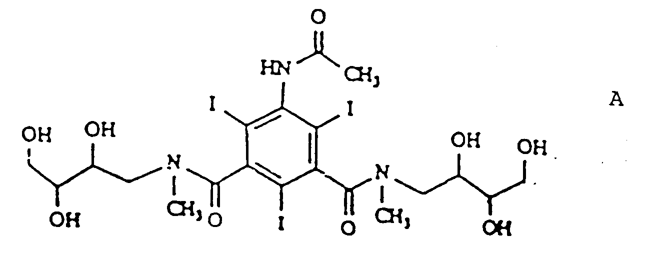

Le composé de formule A peut être préparé de la façon suivante :
a) réduction dans les conditions classiques d'un composé de formule XVI : 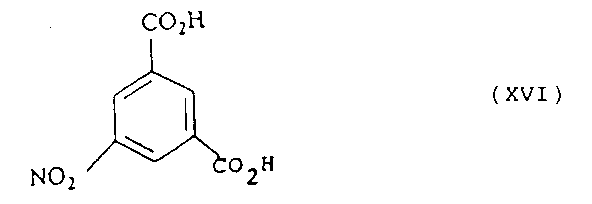 pour obtenir un composé de formule XVII : 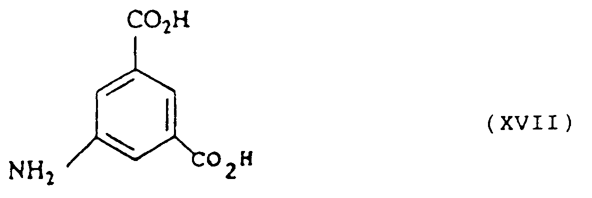
b) iodation du composé de formule XVII pour obtenir le composé de formule XVIII : 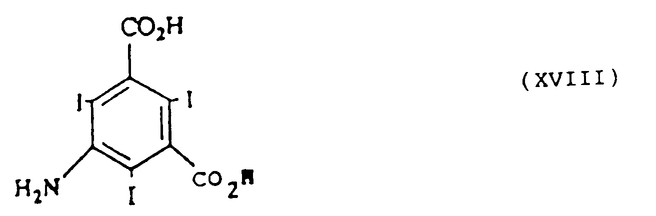
c) chloruration dans les conditions classiques du composé de formule XVIII pour obtenir le composé de formule XIX : 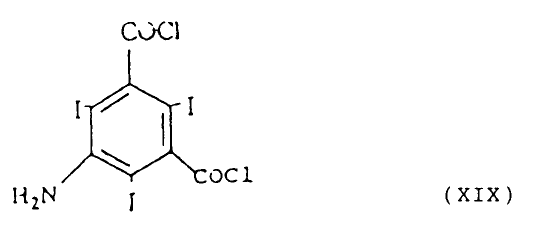
d) acylation du composé de formule XIX dans les condi-tions classiques au moyen d'un composé de formule XII :

   R'₉-CO-Cl (XII)

   dans laquelle R'₉ représente -CH₃, pour obtenir le composé de formule XX : 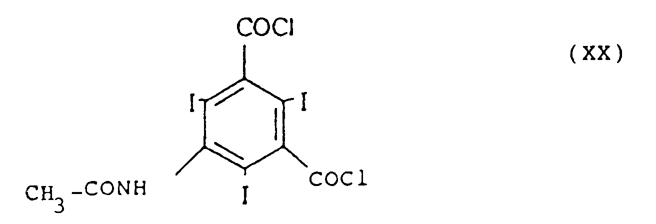
e) amidification du composé de formule XX dans les conditions classiques avec une amine alcool de formule : 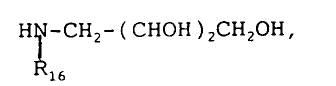 dans laquelle R₁₆ représente -CH₃, de façon à obtenir le composé de formule A :

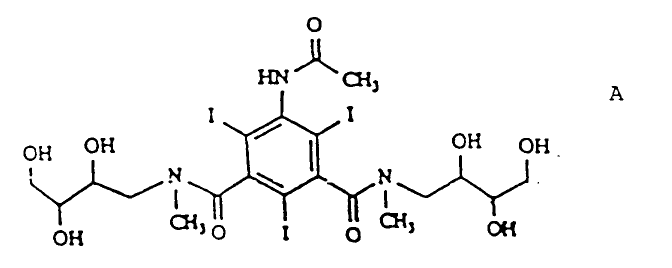

La réaction de l'étape a) est réalisée avantageusement par réduction catalytique par l'hydrogène sur du charbon palladié ou sur du Nickel de Raney ou par réduction chimique dans les conditions classiques.

L'étape d'iodation est effectuée dans les conditions classiques telles que par ICl aqueux ou I₂ en présence de KI/éthylamine ou KICl₂, à des températures variant de 0° C à 100° C.

Les réactions de chloruration sont réalisées de manière usuelle, par exemple au moyen de SOCl₂ ou PCl₅ à chaud.

Les réactions d'acylation sont réalisées avantageusement dans un solvant tel que le DMAC.

Les réactions d'amidification sont effectuées avantageusement en présence de triéthylamine.

La réaction d'acétylation est effectuée de préférence avec l'anhydride acétique dans un solvant en présence de pyridine, de HClO₄, H₂SO₄, DMAP ou avec le chlorure d'acétyle à chaud.

### Préparation de l'amine-alcool intermédiaire

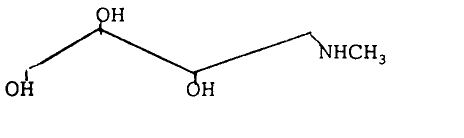

a) Préparation du composé de formule 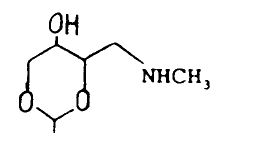
   2 g (13,7 mmoles) d'éthylidène-2,4 D-érythrose obtenu selon le procédé décrit dans J. Am. Chem. Soc. 2301, 1960 Barker R. et al. sont dissous dans 10 cm³ d'eau à 30°C. On ajoute goutte à goutte à 0°C 10 cm³ d'une solution aqueuse de méthylamine (40%). Après retour à la température ambiante, l'agitation est poursuivie pendant 2 h. La solution est ensuite réduite à température ambiante en présence de palladium sur charbon. Le catalyseur est ensuite filtré et le filtrat concentré à sec. Après concrétisation dans l'éther éthylique, 1,7 g de produit du titre sont obtenus, soit un rendement de 77%.
   CCM (dioxanne/H₂O/NH₃:8/3/2) Rf : 0,74
   CCM (CH₂Cl₂/MeOH 8/2) Rf : 0,17.
   RMN¹³C (DMSO) (δ, ppm) 200 MHz 98,2 (C-CH₃); 80,3 (CH-O); 70,5 (CH₂-O); 63,4 (CHOH); 53,1 (CH₂-N); 36,5 (NH-CH₃); 20,7 (C-CH₃).
b) Préparation du composé de formule : 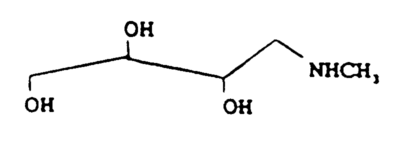
   1,5 g (9,3 mmoles) du produit obtenu en a) sont dissous dans 20 cm³ d'HCl 2N. La solution est agitée à 50°C pendant 5 h. Après concentration, et purification par passage sur résine H⁺, la solution est évaporée à sec. Le résidu est repris dans l'éther éthylique. Après filtration et séchage on obtient 0,8g du produit du titre (Rendement : 64%).
   CCM (dioxanne/H₂O/NH₃:8/3/2) Rf : 0,18
   RMN¹³C (DMSO) (δ,ppm) 200 MHz 74,5 (CH-CH₂OH); 69,6 (CHOHCH₂); 63,3 (CH₂OH); 54,7 (CH₂); 36,12 (NHCH₃) SM (DCI/NH₃) m/z; 153 (M+N⁺H₄); 136 (M+H⁺) pic de base.

Il va de soi que l'invention englobe non seulement le composé de formule A sous forme de mélange racémique mais également les stéréoisomères tels que énantiomères, diastéréoisomères, atropoisomères, isomères SYN-ANTI, ENDO-EXO, E-Z, liés à la présence d'atomes de carbone asymétriques et/ou aux empéchements de rotation dus à l'encombrement stérique apporté par les atomes d'iode et/ou par les substituants R₁, R₂ et R₃ des composés de formule I.

La présente invention a également pour objet des produits de contraste qui comprennent le composé de formule A.

Ces produits de contraste sont utilisés chez l'homme et les animaux à des fins radiologiques.

La forme pharmaceutique préférée des produits de contraste selon l'invention est constituée par des solutions aqueuses des composes.

Les solutions aqueuses contiennent généralement un total de 5 à 100 g d'au moins un composé de formule I pour 100 ml et la quantité injectable de telles solutions peut varier généralement de 1 à 1000 ml.

Les solutions aqueuses du composé de formule A peuvent également contenir certains additifs comme :
- du chlorure de sodium à des concentrations comprises entre 0,1 à 10 mM
- de l'EDTA disodique à des concentrations comprises entre 0,1 et 2 mM
- du citrate de sodium à des concentrations comprises entre 0,1 et 10 mM
- de l'héparine à des doses comprises entre 10 et 100 unités pour 100 ml de solution,
- et des solutions tampons telles que le chlorhydrate de tris(hydroxyméthyl)amino méthane.

Ces compositions peuvent être administrées par toutes voies classiquement utilisées pour les produits de contraste non ioniques iodés. Ainsi elles peuvent être administrées par voie entérale (orale, rectale) ou parentérale (voie intraveineuse, intra-artérielle, intra-articulaire, opacification des cavités) et en particulier dans l'espace sous-arachnoïdien, ainsi que par les voies bronchiques, lymphatique et intra-utérine.

Dans certaines applications particulières, il peut être nécessaire, pour faire le diagnostic d'une pathologie donnée, notamment au niveau d'un organe spécifique, d'avoir recours à ce que l'on appelle une vectorisation de l'agent de contraste qui peut être obtenue par encapsulation dudit agent dans des liposomes, ou par la fixation d'une biomolécule, notamment des protéines.

On donnera ci-après un exemple de composition selon la présente invention.

### Composition

| | |
|---|---|
| Composition de l'exemple 1 | 65 g |
| Eau pour préparation injectable OSP | 100 g |

L'exemple suivant illustre la préparation du composé de formule A.

### EXEMPLE 1

Préparation du composé de formule : (composé A) 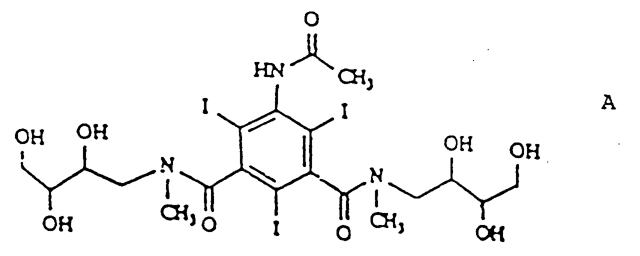 5-acétamido-N,N'-diméthyl-N, N'-bis (2,3,4-trihydroxybutyl)2,4,6-triiodoisophtalamide.

1,2 g (1,85 mmole) de dichlorure de 5-acétamido-2,4,6-triiodoisophtaloyle tel que décrit dans l'exemple 1-3) ci-dessus est additionné à une solution de 1 g (7,4 mmoles) de l'amine-alcool n° 1 préparée selon la méthode décrite précédemment, et de triéthylamine (7,4 mmoles) dans 7 ml de diméthylacétamide. L'agitation est maintenue pendant 45 minutes à 40° C. Le chlorhydrate de triéthylamine est éliminé par filtration.

Le filtrat est évaporé à sec, repris à l'eau et élué sur résines IRN 77 et IRA 67. Après évaporation à sec, on recueille 1,32 g de poudre blanche (Rdt 84 %).
- Dosage d'iode :: 99,5 %
- Pureté HPLC :: 99 % Lichrosphère C18 5 µm NaH₂PO₄ 0,01 M MeOH
- CCM SiO₂ CH₂Cl₂/MeOH :: 70/30, Rf : 0,33, 0,26, 0,58
- dioxane/eau/NH₃ :: 80/30/20, Rf 0,74

RMN¹H (DMSO) 200 MHz (δ, ppm)
(2,1, s, 3H, NHCOCH₃); (2,85, s, 6H N-CH₃); (3,1-4, massifs, 12H, CH et CH₂); (4,3-4,9, massifs, 6H, OH) (10, massifs, 1H, NH)

¹³C (δ, ppm)
(22,28, NHCOCH₃); (37,76 N-CH₃); (50,58 N-CH₂); (62,35, CH₂OH); (69,89 - 73,4, CHOH); (89,45-97,4-98,8, 2C-I) (143,69, Cₐᵣ-N); (148-148,6, Cₐᵣ-CO) (171,9 - 172,1, C=O).

## Revendications

1. Composé non ionique poly-iodé de formule :

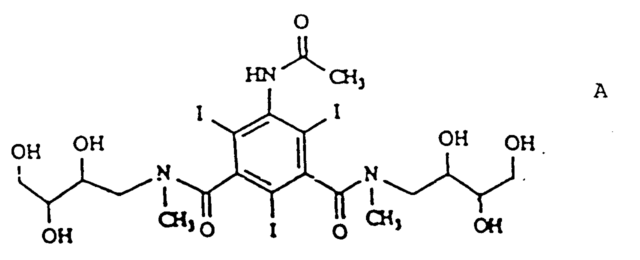

2. Produit de contraste, caractérisé en ce qu'il contient le composé non ionique poly-iodé selon la revendication 1.

3. Produit de contraste selon la revendication 2, caractérisé en ce qu'il est constitué par une solution aqueuse du composé non ionique poly-iodé selon la revendication 1.

## Claims

1. A non-ionic polyiodised compound of the formula:

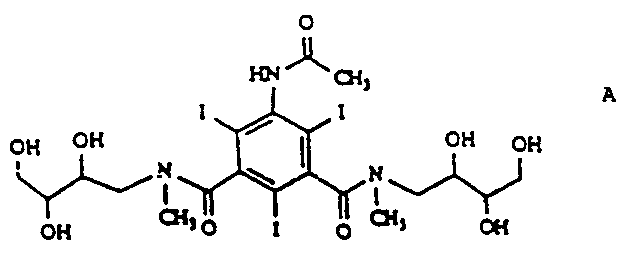

2. A contrast agent, characterised in that it contains the non-ionic polyiodised compound according to claim 1.

3. A contrast agent according to claim 2, characterised in that it comprises an aqueous solution of the non-ionic polyiodised compound according to claim 1.

## Patentansprüche

1. Nichtionische polyiodierte Verbindung der Formel:

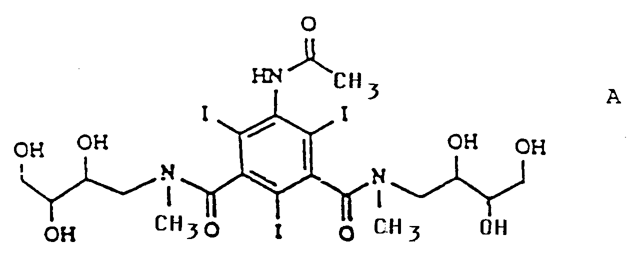

2. Kontrastmittel, **dadurch gekennzeichnet,** daß es die nichtionische polyiodierte Verbindung nach Anspruch 1 enthält.

3. Kontrastmittel nach Anspruch 2, **dadurch gekennzeichnet,** daß es durch eine wässrige Lösung der nichtionischen polyiodierten Verbindung nach Anspruch 1 gebildet ist.
